(19) Europäisches Patentamt
European Patent Office
Office européen des brevets

(11) Veröffentlichungsnummer: **0 024 710**
**A2**

(12) # EUROPÄISCHE PATENTANMELDUNG

(21) Anmeldenummer: **80105016.2**

(22) Anmeldetag: **23.08.80**

(51) Int. Cl.³: **A 61 K 7/13**

(30) Priorität: **01.09.79 DE 2935429**

(43) Veröffentlichungstag der Anmeldung:
**11.03.81 Patentblatt 81/10**

(84) Benannte Vertragsstaaten:
**AT BE CH DE FR GB IT LI NL SE**

(71) Anmelder: **Henkel Kommanditgesellschaft auf Aktien -Patentabteilung- Postfach 1100 Henkelstrasse 67 D-4000 Düsseldorf 1(DE)**

(72) Erfinder: **Konrad, Günther, Dr.**
**Feuerbachweg 12**
**D-4010 Hilden(DE)**

(72) Erfinder: **Lieske, Edgar**
**Hunsrückenstrasse 40**
**D-4000 Düsseldorf(DE)**

(54) **Haarfärbemittel.**

(57) Haarfärbemittel auf Basis von Oxidationsfarbstoffen mit einem Gehalt an 3-Amino-2-methylphenol als Kupplersubstanz und den üblichen Entwicklerkomponenten.

EP 0 024 710 A2

HENKEL KGaA
ZR-FE/Patente
z-sü

P a t e n t a n m e l d u n g

D 6022 EP

"Haarfärbemittel"

Gegenstand der Erfindung sind Mittel zur oxidativen Färbung von Haaren auf Basis von 3-Amino-2-methylphenol als Kupplerkomponente.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen, wie p-Phenylendiaminderivate, Diaminopyridine, 4-Amino-pyrazolonderivate, heterocyclische Hydrazone eingesetzt. Als sogenannte Kupplerkomponenten werden Phenole, Naphthole, Resorcinderivate und Pyrazolone genannt.

Gute Oxidationshaarfarbstoffkomponenten müssen in erster Linie folgende Voraussetzungen erfüllen:

Sie müssen bei der oxidativen Kupplung mit den jeweiligen Entwickler- bzw. Kupplerkomponenten die gewünschten Farbnuancen in austeichender Intensität ausbilden. Sie müssen ferner ein ausreichendes bis sehr gutes Aufziehvermögen auf menschlichem Haar besitzen, und sie sollen darüber hinaus in toxikologischer und dermatologischer

Hinsicht unbedenklich sein. Weiterhin ist von Bedeutung, daß auf dem zu färbenden Haar möglichst kräftige und den natürlichen Haarfarbennuancen weitgehend entsprechende Farbtöne erhalten werden. Ferner kommt der allgemeinen Stabilität der gebildeten Farbstoffe sowie deren Lichtechtheit, Waschechtheit und Thermostabilität ganz besondere Bedeutung zu, um Farbverschiebungen von der ursprünglichen Farbnuance oder gar Farbumschläge in andere Farbtöne zu vermeiden.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, daß Haarfärbemittel auf Basis von Oxidationsfarbstoffen mit einem Gehalt an 3-Amino-2-methylphenol als Kupplersubstanz und den in Oxidationshaarfarben üblichen Entwicklerkomponenten den gestellten Anforderungen in hohem Maße gerecht werden.

Bei dem Einsatz als Kupplerkomponente liefert die erfindungsgemäße Verbindung mit den im allgemeinen für die Oxidationshaarfärbung verwendeten Entwicklersubstanzen sehr intensive und lichtechte, braune bis dunkelblau reichende Farbnuancen und stellt somit eine wesentliche Bereicherung der oxidativen Haarfärbemöglichkeiten dar. Insbesondere mit der Entwicklerkomponente p-Toluylendiamin resultierte eine überaus lichtstabile, interessante rotbraune Haarausfärbung. Aber auch die mit verschiedenen Entwicklerkomponenten erzielbaren dunkelblauen Farbnuancen besitzen erhöhtes Interesse für die Erzeugung natürlicher Brauntöne. Darüber hinaus zeichnet sich 3-Amino-2-methylphenol durch eine gute Löslichkeit in Wasser, eine gute Lagerstabilität und toxikologische sowie dermatologische Unbedenklichkeit aus.

Das erfindungsgemäß als Kupplerkomponente zu verwendende 3-Amino-2-methylphenol stellt eine bekannte Verbindung dar, deren Herstellung von Noelting in den Berichten der Deutschen Chemischen Gesellschaft 37 (1904), Seite 1020/21 beschrieben wird.

Als Beispiele für in den erfindungsgemäßen Haarfärbemitteln einzusetzende Entwicklerkomponenten sind primäre aromatische Amine mit einer weiteren in p-Stellung befindlichen funktionellen Gruppe wie p-Phenylendiamin, p-Toluylendiamin, Monochlor-p-phenylendiamin, p-Dimethylaminoanilin, p-Aminophenol, p-Diaminoanisol bzw. andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1 - 4 Kohlenstoffatomen darstellt, ferner Diaminopyridinderivate, heterocyclische Hydrazonderivate, 4-Aminopyrazolonderivate, wie 4-Amino-1-phenyl-3-carbamoylpyrazolon-5 anzuführen.

Als weitere Entwicklerkomponenten, die sich mit Vorteil mit den Benzimidazolderivaten kombinieren lassen, sind Tetraaminopyrimidine der allgemeinen Formel

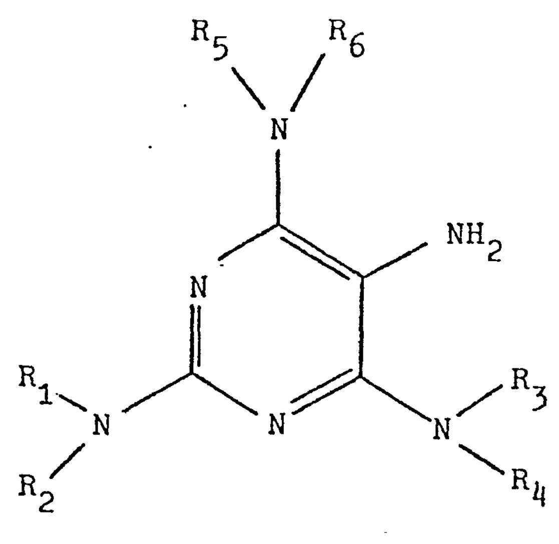

/4

in der $R_1$ - $R_6$ Wasserstoff, einen Alkylrest mit 1 - 4 Kohlenstoffatomen, den Rest $-(CH_2)_n-X$, in dem n = 1 - 4 und X eine Hydroxylgruppe, ein Halogenatom, eine $-NH_2-$, -NHR'- und -NR'R"-Gruppe sein können, wobei R' und R" Alkylreste mit 1 - 4 Kohlenstoffatomen bedeuten können oder mit dem Stickstoffatom zu einem heterocyclischen Ring, der ein weiteres Stickstoffatom oder Sauerstoffatom enthalten kann, geschlossen sind, $R_1$ und $R_2$, bzw. $R_3$ und $R_4$, bzw. $R_5$ und $R_6$ mit dem jeweiligen Stickstoffatom einen heterocyclischen 5- oder 6-gliedrigen Ring mit einem oder zwei Stickstoffatomen oder einem Stickstoffatom und einem Sauerstoffatom darstellen können sowie deren anorganische oder organische Salze zu nennen.

Die als Entwicklerkomponenten zu verwendenden Tetraaminopyrimidine können entweder als solche oder in Form ihrer Salze mit anorganischen oder organischen Säuren, wie z. B. als Chloride, Sulfate, Phosphate, Acetate, Propionate, Lactate, Citrate eingesetzt werden.

Für die Kombination mit dem erfindungsgemäß als Kupplerkomponente zu verwendendem 3-Amino-2-methylphenol geeignete Entwicklersubstanzen stellen zum Beispiel
2,4,5,6-Tetraamino-,
4,5-Diamino-2,6-bismethylamino-,
2,5-Diamino-4,6-bismethylamino-,
4,5-Diamino-6-butylamino-2-dimethylamino-,
2,5-Diamino-4-diethylamino-6-methylamino-,
4,5-Diamino-6-diethylamino-2-dimethylamino-,
4,5-Diamino-2-diethylamino-6-methylamino-,
4,5-Diamino-2-dimethylamino-6-ethylamino-,
4,5-Diamino-2-dimethylamino-6-isopropylamino-,
4,5-Diamino-2-dimethylamino-6-methylamino-,
4,5-Diamino-6-dimethylamino-2-methylamino-,
4,5-Diamino-2-dimethylamino-6-propylamino-,

2,4,5-Triamino-6-dimethylamino-,
4,5,6-Triamino-2-dimethylamino-,
2,4,5-Triamino-6-methylamino-,
4,5,6-Triamino-2-methylamino-,
4,5-Diamino-2-dimethylamino-6-piperidino-,
4,5-Diamino-6-methylamino-2-piperidino-,
2,4,5-Triamino-6-piperidino-,
2,4,5-Triamino-6-anilino-,
2,4,5-Triamino-6-benzylamino-,
2,4,5-Triamino-6-benzylidenamino-,
4,5,6-Triamino-2-piperidino-,
2,4,6-Trismethylamino-5-amino-,
2,4,5-Triamino-6-di-n-propylamino-,
2,4,5-Triamino-6-morpholino-,
2,5,6-Triamino-4-dimethylamino-,
4,5,6-Triamino-2-morpholino-,
2,4,5-Triamino-6-β-hydroxyethylamino-,
4,5,6-Triamino-2-β-amino-ethylamino-,
2,5,6-Triamino-4-β-methylamino-ethylamino-,
2,5-Diamino-4,6-bis-γ-diethylamino-propylamino-,
4,5-Diamino-2-methylamino-6-β-hydroxy-ethylamino-,
5-Amino-2,4,6-triethylamino-,
2,4-Bis-β-hydroxyethylamino-6-anilino-5-amino-pyrimidin
dar.

In den erfindungsgemäßen Haarfärbemitteln wird die Kupplerkomponente im allgemeinen in etwa molaren Mengen, bezogen auf die verwendeten Entwicklersubstanzen, eingesetzt. Wenn sich auch der molare Einsatz als zweckmäßig erweist, so ist es jedoch nicht nachteilig, wenn die Kupplerkomponente in einem gewissen Überschuß oder Unterschuß zum Einsatz gelangt. Es ist ferner nicht erforderlich, daß die Entwicklerkomponente und die Kupplersubstanz

einheitliche Produkte darstellen, vielmehr kann die Entwicklerkomponente Gemische der erfindungsgemäß zu verwendenden Entwicklerverbindungen darstellen. Darüber hinaus können die erfindungsgemäßen Haarfärbemittel gegebenenfalls übliche direktziehende Farbstoffe im Gemisch enthalten, falls dies zur Erzielung gewisser Farbnuancen erforderlich ist.

Die oxidative Kupplung, das heißt die Entwicklung der Färbung, kann grundsätzlich wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Die erfindungsgemäßen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwickler-Kombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind zum Beispiel Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose,

/7

Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie zum Beispiel Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8 bis 10 erfolgen. Die Anwendungstemperaturen bewegen sich dabei im Bereich von 15 bis $40^{o}$ C. Nach einer Einwirkungsdauer von ca. 30 Minuten wird das Haarfärbemittel vom zu färbenden Haar durch Spülen entfernt. Hernach wird das Haar mit einem milden Shampoo nachgewaschen und getrocknet.

Die nachfolgenden Beispiele sollen den Erfindungsgegenstand näher erläutern, ohne ihn jedoch hierauf zu beschränken.

/8

## B e i s p i e l e

Das in den nachfolgenden Beispielen als Kupplerkomponente einzusetzende 3-Amino-2-methylphenol wurde entsprechend den Angaben von Noelting in Berichte der Deutschen Chemischen Gesellschaft 37 (1904), Seite 1020/21 hergestellt.

Als Entwicklerkomponenten wurden in den folgenden Beispielen die nachstehend genannten Verbindungen eingesetzt:

E 1: p-Toluylendiamin
E 2: p-Phenylendiamin
E 3: p-Diaminoanisolsulfat
E 4: N,N-Dimethyl-p-phenylendiamin
E 5: p-Aminophenol
E 6: 2-Chlor-p-phenylendiamin
E 7: 1-Phenyl-3-carbamoyl-4-amino-pyrazolon-5
E 8: N-Methyl-pyrrolidon-2-hydrazon
E 9: N-Butyl-N-sulfobutyl-p-phenylendiamin
E 10: N-Methyl-p-phenylendiamin
E 11: $N^4$,$N^4$-Diethyl-2-methyl-p-phenylendiamin
E 12: N-Ethyl-N-hydroxyethyl-p-phenylendiamin
E 13: 2,4,5,6-Tetraaminopyrimidin.

Die erfindungsgemäßen Haarfärbemittel wurden in Form einer Cremeemulsion eingesetzt. Dabei wurden in eine Emulsion aus

10 Gewichtsteilen Fettalkoholen der Kettenlänge $C_{12}-C_{18}$,
10 Gewichtsteilen Fettalkoholsulfat (Natriumsalz) der Kettenlänge $C_{12}-C_{18}$,
75 Gewichtsteilen Wasser

jeweils 0,01 Mol der in der nachstehenden Tabelle aufgeführten Entwicklersubstanzen und 3-Amino-2-methylphenol eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde mit 1 %iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit zusätzlichem Oxidationsmittel wurde auf zu 90 % ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

/10

## T a b e l l e 1

| Beispiel | Entwickler | Erhaltener Farbton mit 1 %iger $H_2O_2$-Lösung |
|---|---|---|
| 1 | E 1 | rotbraun |
| 2 | E 2 | braunviolett |
| 3 | E 3 | dunkelblau |
| 4 | E 4 | dunkelblau |
| 5 | E 5 | kastanienbraun |
| 6 | E 6 | dunkelbraun |
| 7 | E 7 | dunkelviolett |
| 8 | E 8 | gelbbraun |
| 9 | E 9 | blaugrau |
| 10 | E 10 | dunkelblau |
| 11 | E 11 | dunkelblau |
| 12 | E 12 | dunkelblau |
| 13 | E 13 | dunkelviolett |

"Haarfärbemittel"

Patentansprüche:

1. Haarfärbemittel auf Basis von Oxidationsfarbstoffen, gekennzeichnet durch einen Gehalt an 3-Amino-2-methylphenol als Kupplersubstanz und den in Oxidationsfarben üblichen Entwicklerkomponenten.

2. Haarfärbemittel nach Anspruch 1, gekennzeichnet durch einen Gehalt an p-Toluylendiamin als Entwicklerkomponente.

3. Haarfärbemittel nach Anspruch 1 und 2, gekennzeichnet durch einen Gehalt an Entwickler-Kuppler-Kombinationen von 0,2 bis 5 Gewichtsprozent, vorzugsweise 1 bis 3 Gewichtsprozent, bezogen auf das gesamte Mittel.